# EUROPEAN PATENT SPECIFICATION

(11) **EP 1 549 386 B1**
(45) Date of publication and mention of the grant of the patent: **14.04.2010**
(21) Application number: 03767124.5
(22) Date of filing: 01.08.2003
(51) Int. Cl.: A61N 1/36

(54) **APPARATUS FOR HEMODYNAMIC-BASED OPTIMIZATION OF CARDIAC PACING**
GERÄT ZWECKS HÄMODYNAMISCHBASIERTER OPTIMIERUNG DER KARDIALEN STIMULATION
APPAREIL POUR L'OPTIMISATION HEMODYNAMIQUE DE LA STIMULATION CARDIAQUE

(30) Priority: 02.08.2002 US 400796 P
(43) Date of publication of application: 06.07.2005
(73) Proprietor: Medtronic, Inc., Minneapolis, MN 55432-5604 (US)
(72) Inventor: BRAUNSCHWEIG, Frieder, S-17176 Stockholm (SE); KJELLSTROM, Barbro, Minneapolis, MN 55405 (US)
(74) Representative: Hughes, Andrea Michelle
(86) International application number: PCT/US2003/024292
(87) International publication number: WO 2004/012808

(56) References cited:
- US-A- 5 421 830
- US-A- 5 891 176

## Description

The present invention relates to the field of implantable medical devices. In particular, the present invention discloses apparatus for optimizing cardiac pacing algorithms based on hemodynamic physiologic data collected using a hemodynamic transducer implanted in a pacemaker patient. The present invention has specific utility with respect to heart failure patients suffering from related chronic symptoms.

Cardiac resynchronization therapy (CRT) has gained increased use as an alternative treatment in patients with drug refractory heart failure and an intraventricular conduction delay. Current bi-ventricular pacemakers offer a number of programmable parameters that have potential impact on the hemodynamic status, such as heart rate, A-V- and V-V-interval and pacing mode. In patients with compromised central hemodynamics, optimization of pacemaker algorithms may be crucial for the treatment success of resynchronization. Echocardiography and Doppler techniques are commonly used to optimize the A-V-delay based on measurements of the diastolic mitral inflow pattern or the aortic time velocity integral. However, echocardiography equipment is not always easily accessible for the physician and the usefulness of a short-term evaluation of hemodynamic parameters at rest for long-term pacemaker programming in ambulatory patients is disputed.

Continuous hemodynamic monitoring with an implanted device is technically feasible, safe and delivers accurate measurements over time. Initial studies suggest that such hemodynamic monitoring may help to tailor diuretic and other drug treatments in patients with chronic severe heart failure.

With respect to pressure sensing apparatus capable of chronic *in vivo* operation, many devices and methodologies have been proposed and/or implemented in the prior art. In this regard, the following issued U.S. patents provide added details for several representative pressure monitoring techniques; namely: U.S. Pat. Nos. 5,368,040; 5,564,434;.6,171,252; 6,221,024 and US 5,626,623.

U.S. 5,421,830 discloses a programming system that allows a physician to optimize the settings of arrhythmia detection criteria and/or parameters related to hemodynamic performance to be programmed into an implanted cardiac stimulating device.

The inventors propose that an implantable pulse generator (IPG) having an integrated hemodynamic sensor coupled thereto as defined in claim 1 provides individual hemodynamically-optimized IPG interval timing; particularly for patients suffering from the debilitating effects of heart failure. The present invention is described with respect to test results obtained by observing a heart failure patient suffering from end-stage heart failure. Two discrete devices, an IPG, in this case an implanted bi-ventricular pacemaker, and a hemodynamic monitor, were chronically implanted. Specifically, the study evaluated whether the hemodynamic monitor could be used for optimization of the A-V-delay and heart rate (HR).

The present invention demonstrates that continuous hemodynamic monitoring can be used to identify the optimal A-V delay in a pacemaker implanted patient with end-stage heart failure (HF). The A-V-delay determines the timing of late diastolic filling in relation to the onset of ventricular contraction and the duration of diastolic filling. An optimal tuning of the A-V-delay improves left ventricular filling pressures in patients with a DDD-programmed pacemaker and is particularly important in the presence of a compromised left ventricular function. The inventors recognized that the lowest estimated pulmonary artery diastolic (ePAD) pressure, an indirect parameter of the left ventricular end-diastolic pressure, is an indicator for the optimal A-V interval. Importantly, measurements of the ePAD provided the same optimal A-V-delay as echocardiographic assessment of left ventricular diastolic filling by standard echocardiographic methods (using the so-called Ritter method for adult transthoracic echocardiography).

By practicing the present invention, the HR determined as optimal during the acute hemodynamic test performed in a clinical setting did *not* turn out to be optimal when implemented for ambulatory patients performing the activities of daily living (ADL): In the acute test a decrease of ePAD and RVDP was observed simultaneously with an increase of RVPP and maximal dP/dt at a heart rate of 90 bpm. The present invention demonstrates that continuous hemodynamic monitoring provides useful information for the optimization of hemodynamically important pacemaker algorithms such as the A-V-delay,heart rate and pacing mode. In contrast to echocardiography, hemodynamic monitoring offers the potential to adjust pacemaker parameters even under the condition of exercise or during ADL. In patients with heart failure, the hemodynamic information may also be used to guide drug treatment and volume management.

The present invention may be used to operate cardiac stimulation devices, especially those specifically designed for use by HF patients, that include pacing intervals capable of providing hemodynamic optimization. For example, the techniques of the present invention may be applied to enhance hemodynamics using a traditional dual-chamber pacemaker, a triple-chamber, bi-ventricular CRT device, an implantable cardioverter-defibrillator (ICD), and the like. In one form of the present invention a cardiac stimulation device communicates with a hemodynamic monitoring sensor to optimize pacing intervals. In another form of the present invention, a unitary HF management device with programmable cardiac stimulation capabilities includes an integrated hemodynamic measurement system. Other forms of the present invention and innovative aspects thereof will become readily apparent to those of skill in the art upon review of the present disclosure. For example, while viewed as an inherent aspect of the invention, the present invention may be implemented as computer readable instructions stored on a computer readable medium that are executed by a microprocessor-based implantable or external cardiac stimulation device.

Figure 1 depicts measured hemodynamic impact of different A-V-delays during bi-ventricular pacing at a heart rate of 70 bpm (mean and SD of five consecutive tests).

Figure 2 is a table depicting various hemodynamic metrics for certain pacing intervals and heart rates.

Figure 3 depicts continuous hemodynamic monitoring during 7 weeks at different back-up heart rates in a patient with a bi-ventricular pacemaker. Median (dark line) 6^{th} and 94^{th} percentile (light line) of Right ventricular (RV) systolic pressure (RVSP) RV diastolic pressure (RVDP), RV pulse pressure (RVPP) and RV contraction velocity (RV dP/dt). The heart rate (HR) is represented by the dotted line.

The present invention shall be described with reference to an exemplary patient who received cardiac pacing therapy according to one form of the invention. The exemplary patient presented with well-known cardiovascular risk factors that included cigarette smoking, hypertension and a family history of coronary artery disease (CAD) and HF. Prior to receiving cardiac pacing therapy according to the present invention, the patient suffered from an infero-lateral myocardial infarction (MI) that was treated by thrombolysis. Following the MI episode, an echocardiography examination exposed a moderately enlarged left ventricle (LV) with a left ventricular ejection fraction (LVEF) of approximately 45%. Following the echocardiography examination, a complete revascularization by coronary artery by-pass grafting (CABG) was performed. In the post surgery period symptoms of severe HF appeared and the LVEF decreased to approximately 15-20%. Drug therapy with diuretics, enalapril, carvedilol, ASA, pravastatin and digoxin led to significant clinical improvement. Later, the patient was included in a clinical trial conducted on behalf of Medtronic, Inc. of Minneapolis, Minnesota, U.S.A. (for the Chronicle® implantable hemodynamic monitor). This trial was a study to evaluate the technical accuracy and reliability of an implantable hemodynamic monitor (IHM) over time. The corresponding IHM information revealed an episode of paroxysmal atrial fibrillation (AF) and anticoagulant treatment (with warfarin) was initiated. During the following eight months, three hospitalizations occurred for a troponin-positive, acute coronary syndrome caused by the paroxysmal AF. Following successful cardioversion of the AF, the clinical status of the patient deteriorated steadily. Following the hospitalizations, echocardiography measurements showed significantly enlarged ventricles with a left ventricular end-diastolic diameter (LVEDD) of 81 mm, LVEF of about 10%, mitral insufficiency (grade ²/₄) and tricuspid regurgitation (grade ³/₄). Due to the significant remodeling of the ventricles, especially the LV, and the declining HF status of the patient, heart transplantation was the next scheduled procedure.

Due to symptomatic bradycardia, first-degree heart block (P-Q interval of 260 ms) and a left anterior hemi-block with a QRS duration of 120 ms, a bi-ventricular pacemaker was implanted (the InSync® brand pacemaker manufactured by Medtronic, Inc.). This bi-ventricular pacemaker provides a variety of programmable pacing intervals (e.g., an A-V interval, a V-V interval, paced-A-V or "PA-V" interval, and sensed-A-V or "SA-V" interval, and the like).

The Chronicle^{®} brand IHM (also manufactuered by Medtronic, Inc. as the Model 9520) allows continuous, ambulatory hemodynamic recording using a pressure sensor placed in the right ventricular (RV) outflow tract. This IHM measures HR, activity and several pressure or pressure related parameters that are stored in memory of the IHM circuitry. The data collection can be programmed to various follow-up periods that regulate the storage interval of the parameters and the stored data may be telemetrically conveyed to remote devices. The IHM device is capable of providing measurements (and storing): RV systolic pressure (RVSP), RV diastolic pressure (RVDP), estimated pulmonary artery diastolic (ePAD) pressure, rate-of-change pressure (dP/dt) and HR both acutely (nominal storage interval of two seconds) and ambulatory (nominal storage interval of six minutes).

Hemodynamic information from the IHM was collected at rest during test protocols including eight paced A-V (PA-V) intervals (110-250 ms) and seven different HR (50-110 bpm). A-V-intervals were tested at a HR of 70 bpm and HR at an PA-V interval delay of 180 ms and a SA-V interval delay of 130 ms. The different A-V-intervals and HR were programmed in a randomized order for 1-2 minutes each and a median of the last 30 seconds was used for the data analysis. The protocol was repeated each week over five consecutive weeks. Echo-Doppler measurements were used to assess the diastolic mitral inflow pattern. According to the Ritter method of echocardiography, the A-V-delay providing complete end-diastolic filling without shortening of the diastolic filling time was considered optimal. In addition, the hemodynamic impact of four different heart rates (60-90 bpm) were tested during periods of 5-14 days each during ADL.

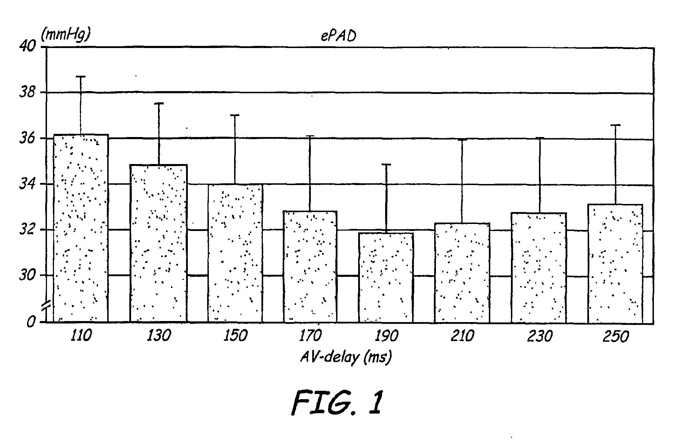

Referring now to Figure 1, an optimal A-V delay was determined as 190 ms (PA-V interval) and 140 ms (SA-V interval) using the lowest obtained ePAD from the IHM. At the optimal A-V-interval the mean ePAD was 4.2 mrnHg(559.9 Pa) lower (31.9 mmHg (4.3 kPa) compared to the poorest setting (36.1 mmHg (4.8 kPa) at 110 ms). The same PA-V interval of 190 ms was determined optimal by the Echo-Doppler measurements. As will be appreciated by those of skill in the art, one technique for practicing the present invention involves iteratively applying one of a set of intervals for a sufficient period of time for the hemodynamic effects of the interval to stabilize.
Then, at least one discrete pressure measurement is stored to a memory structure, such as a look up table (LUT). Of course, while a LUT can be used to store each applied pacing interval and corresponding hemodynamic data, other computer readable storage media may be used. For example, as is known to those of skill in the art, serial access memory (SAM) buffers, random access memory (RAM) including dynamic and static variants thereof (DRAM, SRAM), and read only memory (ROM) also known as "firmware," and programmable and electrically erasable programmable variants thereof (PROM, EEPROM also known as "flash memory") and the like may be successfully used in practicing the present invention. Once a desired range of intervals have been applied and the resultant hemodynamic measurements suitably stored, a comparison of the intervals and measurements provides the optimized settings for a programmable IPG. Based on the data set forth in Figure 1, it appears that at the shortest A-V delay inteval of 110 ms an ePAD pressure of approximately 36 mmHg (4.8 kPa) was developed. The ePAD pressure was reduced from this value, to slightly less than 35 mmHg (4.7 kPa) for an A-V delay interval of 130 ms, to approximately 34 mmHg (4.5 kPa) for an A-V delay interval of 150 ms, to about 33 mmHg (4.44 kPa) for an A-V delay interval of 170 ms. For an A-V delay interval of 190 ms an ePAD pressure of approximately less than about 32 mmHg (4.3 kPa) was measured. As depicted in Fig. 1, the A-V delay interval provided the lowest ePAD pressure as for A-V delay intervals of 210 ms, 230 ms and 250 ms the ePAD pressure sequentially increased. Of course, while Fig. 1 depicts a nominal 20 ms separation between successive ePAD pressure measurements (from 110 ms to 250 ms A-V delay intervals), any suitable range and magnitude of separation may be used for a given patient. In addition, assuming adequate transducer tolerance, calibration and/or sensitivity, once the fiducial point is reached (i.e., 190 ms A-V delay interval in Figure 1) a second, third or additional round of optimization may occur perferably using smaller increments and a shorter overall range to more rapidly arrive at the hemodynamically optimized values. For example, given the data set forth in Figure 1, another optimization iteration in 5 ms or 10 ms steps over a range of about 170 ms to about 210 ms should provide an even more optimal pacing interval setting.

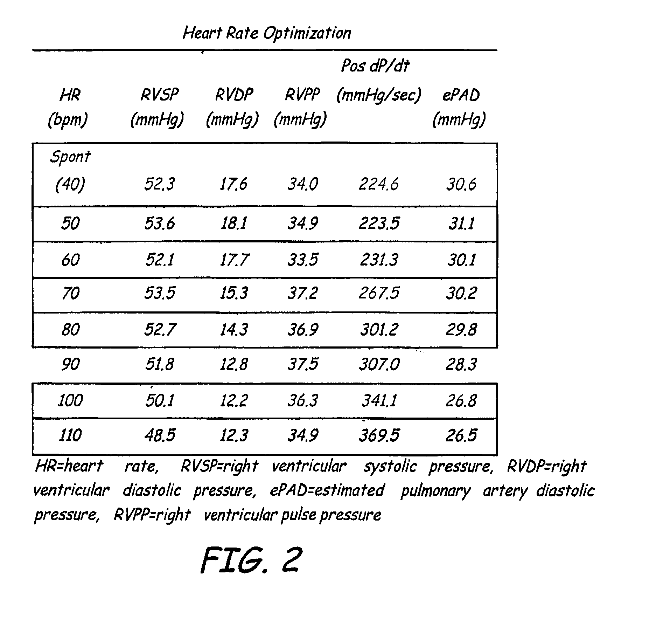

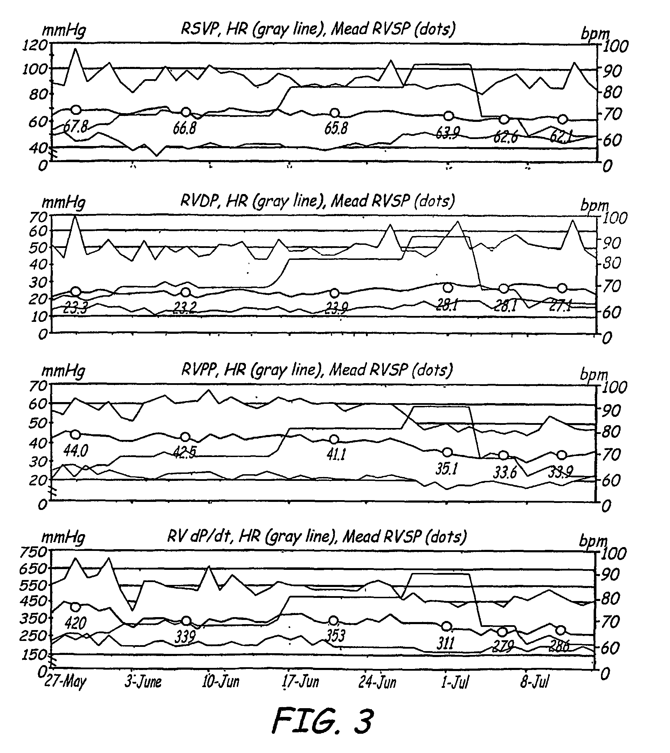

Referring now to the table set forth as Figure 2, during the acute, clinical test of various HR an increase in right ventricular pulse pressure (RVPP) and a decrease in right ventricular diastolic pressure (RVDP) and ePAD was obseved when HR was programmed to 90 bpm. Thus, as compared to a spontaneous, or intrinsic, HR of 40 bpm and paced HR from 50 bpm to 110 bpm, the overall combination of discrete pressure measurements (and derivatives thereof) appear optimal at 90 bpm. For example, upon inspection of Figure 2 it is apparent that RVSP has nearly a maximum amplitude, RVDP has a near minimal amplitude, RVPP has the maximum amplitude, ePAD pressure has near-minimum value, and the maximum rate of pressure change (i.e., dP/dtₘₐₓ) is one of the highest for each HR tested. As is known in the art, the combination of the discrete pressure measurements (and derivatives thereof) resulting from a 90 bpm HR are qualitatively superior to the pressure data resulting from each of the other HR values tested. In summary, based on the pressure measurements for a range of HR values from 50 bpm to 100 bpm a HR value of 90 bpm was found optimal. However, as depicted in Figure 3A - 3D, in an ambulatory setting with substantially continuous hemodynamic pressure monitoring, hemodynamic deterioration was indicated by increased dP/dt when HR was programmed above 70 bpm.

That is, as depicted at Figure 3A, the RVSP, HR and Mean RVSP are plotted for the time period 27 May to about 15 July and from about 17 June to 1 July the HR was programmed above 70 bpm. During this period of relatively elevated HR the RVSP measurements were depressed relative to the rest of the measurements (when HR remained at or near about 70 bpm).

Also, as depicted at Figure 3B, the RVDP, HR and Mean RVDP are plotted for the time period 27 May to about 15 July and from about 17 June to 1 July the HR was programmed above 70 bpm. During this period of relatively elevated HR the RVDP measurements were elevated relative to the rest of the measurements (when HR remained at or near about 70 bpm).

Furthermore, as depicted at Figure 3C, the RVPP, HR and Mean RVPP are plotted for the time period 27 May to about 15 July and from about 17 June to 1 July the HR was programmed above 70 bpm. During this period of relatively elevated HR the RVSP measurements were depressed relative to the rest of the measurements (while HR remained at or near about 70 bpm). Finally, as depicted at Figure 3D, the RV dP/dt, HR and Mean RV dP/dt are plotted for the time period 27 May to about 15 July and from about 17 June to 1 July the HR was programmed above 70 bpm. During this period of relatively elevated HR the RV dP/dt measurements were relatively depressed when compared to the rest of the measurements (while HR remained at or near about 70 bpm).

The present disclosure provides support for the proposition that continuous hemodynamic monitoring can be used to identify the optimal A-V-delay in a pacemaker patient suffering from end-stage heart failure. The A-V-delay determines the timing of late diastolic filling in relation to the onset of ventricular contraction and the duration of diastolic filling. An optimal tuning of the A-V-delay improves left ventricular filling pressures in patients with a DDD-programmed pacemaker and is particularly important in the presence of a compromised left ventricular function. Therefore, the lowest ePAD pressure, an indirect parameter of the left ventricular end-diastolic pressure, was used as a primary indicator for the optimal A-V interval. The measurements of the ePAD pressure illustrates that the same optimal A-V-delay as echocardiographic assessment of left ventricular diastolic filling by the standard Ritter method. The HR determined as optimal during the acute hemodynamic test did not turn out to be optimal during performance of ADL. In an acute clinical test, a decrease of ePAD pressure and RVDP was observed simultaneously with an increase of RVPP and maximal dP/dt at a heart rate of 90 bpm. However, while ambulatory (i.e., performing ADL), a HR programmed above 70 bpm had the opposite effect. That is, increases in ePAD pressure and RVDP and lowering of RVPP and lowering of maximal dP/dt. This indicates that an "optimal" HR as determined by a test protocol performed in a clinical setting by a resting patient may help to acutely improve hemodynamics, for example in the situation of acutely de-compensated heart failure patient where an increase in HR is required to improve cardiac output (CO), but will not provide chronic relief for an ambulatory HF patient.

According to the teaching of the present invention, optimal ambulatory HR and pacing interval programming may only be determined during exercise or while performing ADL as opposed to relatively static, acute clinically-derived HR and pacing interval values. In addition to the exemplary and illustrated example utilized herein, the techniques of the present invention may be used to optimize hemodynamics resulting from different V-V-delay intervals (i.e., the interval between stimulation of the right and left ventricle) in a bi-ventricular CRT pacing modality. Also, the present invention can be used to provide hemodynamically-optimized HR and pacing interval programming for a variety of different pacing modes (for example bi-ventricular vs. left ventricular pacing).

As previously mentioned, according to one form of the present invention two devices may be utilized, an IPG and an IHM (or external variants thereof) and in a second form of the invention requires a single medical device having both pulse generator and hemodynamic monitoring capabilities. In the event that two devices are utilized to practice the present invention, one or both may be external to a patient and either may be implemented in advance of the other device. For example, an IHM may be implanted first and a bi-ventricular pacemaker may be implanted later. Accordingly, a hemodynamic sensor integrated with (or within) a bi-ventricular pacemaker or an ICD may be implemented according to the present invention as an integrated heart failure management device. Such a device allows for recording both long-term hemodynamic trends that will help improve overall cardiac therapy outcomes for a patient, as well as for the customized hemodynamic tuning of the pacemaker/ICD operational parameters on a patient-by-patient basis.

As is known in the art, direct telemetric communication between the two devices may be used to convey and/or store pacing interval and hemodynamic information. In addition, known mass data collection, processing and distribution techniques may be used under the so-called patient management paradigm involving all forms of data transmission (e.g., internet telephony, wireless communication, etc.).

In contrast to echocardiography visualization techniques (according to Ritter et al.), sensor-based HR and pacing interval optimization procedures may be performed automatically or upon command using a suitably programmed processor-based medical device. Also, a clinician may at any time practice the techniques of the present invention using remote communication and monitoring systems even when the patient is present only telemetrically. Toward that end, the various data telemetry and remote patient management technologies of Medtronic, Inc. may be readily applied so that such optimization can be simply, efficiently and quickly administered irrespective of patient location.

The present invention provides an integrated hemodynamic sensor for optimization of IPG function according to hemodynamic response during stress and during performance of the ADL. In addition, such an integrated hemodynamic sensor provides for automatic, short-term adjustments after changes of the patient's clinical condition as well as closed-loop programming of pacing intervals and heart rate based at least in part upon essentially real-time hemodynamic measurements.

The present invention demonstrates that continuous hemodynamic monitoring provides useful method, structure and therapy delivery processes for the optimization of hemodynamicly important IPG operating parameters such as the A-V-delay, heart rate and pacing mode. In contrast to echocardiography, hemodynamic monitoring offers the potential to adjust pacemaker parameters even during exercise or during performance of ADL. In patients suffering the deleterious effects of HF, the hemodynamic information, including hemodynamic trend information, may also be used to guide drug treatment and volume management. Therefore, future devices designed for the use in patients with heart failure, such as traditional dual chamber pacemakers, bi-ventricular CRT devices, ICDs, and the like that contain a hemodynamic monitoring sensor will constitute an integrated HF management device.

## Claims

1. A system for collecting hemodynamic data from a patient and utilizing said data to optimize a cardiac pacing regimen for said patient, **characterized by**:
a hemodynamic monitor means arranged to collect hemodynamic data during a period of time when a heart rate of the patient is elevated above a resting rate due to activity of said patient and for storing said collected hemodynamic data, wherein said hemodynamic data consists of a lowest estimated pulmonary artery diastolic pressure (ePAD) value;
means for monitoring and/or stimulating cardiac tissue of a patient to provide or restore a desired cardiac rhythm; and
means for integrating the collected lowest estimated pulmonary artery diastolic pressure (ePAD) value with the means for monitoring and/or stimulating cardiac tissue to optimize an atrio-ventricular (AV) pacing interval for said patient.

2. A system according to claim 1, wherein the hemodynamic monitor means comprises one of the following transducers, each of which provides an output signal directly or indirectly indicative of at least one hemodynamic metric of the patient:
an absolute pressure sensor adapted to be fluidly coupled to a cardiac chamber of the patient, an absolute pressure sensor adapted to be fluidly coupled to a pulmonary artery of a patient, an absolute or a differential pressure sensor adapted to be fluidly coupled to a portion of the vasculature of a patient.

3. A system according to claim 1, wherein the means for monitoring and/or stimulating comprises one of the following:
a pulse generator, an implantable pacemaker, an implantable cardioverter defibrillator, a muscle stimulation apparatus, an external pacemaker.

4. A system according to claim 3, wherein the hemodynamic monitor means comprises one of the following:
an absolute pressure sensor adapted to be fluidly coupled to a cardiac chamber of the patient, an absolute pressure sensor adapted to be fluidly coupled to a pulmonary artery of a patient, a differential pressure sensor adapted to be fluidly coupled to a portion of the vasculature of a patient, an implantable absolute pressure sensor coupled to an external reference pressure signal; and
wherein an activity-level measurement means is optionally coupled to said patient and an output signal of said activity-level measurement means is time-synchronized to the hemodynamic monitor means and said activity-level measurement means is derived from an accelerometer transducer or a piezoelectric crystal transducer.

5. A computer readable medium containing instructions which, when run on a system as claimed in any of claims 1 to 4, cause the system to perform a method for optimizing hemodynamics of a patient using hemodynamic data collected from said patient, comprising:
instructions for collecting hemodynamic data consisting of a lowest estimated pulmonary artery diastolic pressure (ePAD) value from said patient, during a period of time when a heart rate of the patient is elevated above a resting rate due to activity by said patient, with a hemodynamic monitor adapted to be disposed in fluid contact with a volume of venous blood of said patient;
instructions for storing said hemodynamic data in a computer readable memory medium;
instructions for collecting cardiac event data from the patient;
instructions for storing the cardiac event data in a computer readable memory medium;
instructions for analyzing said lowest estimated pulmonary artery diastolic pressure (ePAD) value in conjunction with said cardiac event data to determine a cardiac stimulation sequence intended to optimize an atrio-ventricular (AV) pacing interval of said patient; and
instructions for providing said cardiac stimulation sequence to an implantable cardiac rhythm stimulation and/or monitoring device.

## Patentansprüche

1. System zum Sammeln von hämodynamischen Daten von einem Patienten und zum Verwenden dieser Daten zum Optimieren eines Herzstimulationsregimes für den Patienten, **gekennzeichnet durch**:
hämodynamische Überwachungsmittel, die dafür eingerichtet sind, hämodynamische Daten während einem Zeitraum zu sammeln, in dem eine Herzfrequenz des Patienten aufgrund einer Aktivität des Patienten eine Ruhefrequenz überschreitet, und zum Speichern der gesammelten hämodynamischen Daten, wobei die hämodynamischen Daten aus einem geringsten abgeschätzten pulmonalarteriellen diastolischen Druckwert (ePAD) bestehen;
Mittel zum Überwachen und/oder Stimulieren von Herzgewebe eines Patienten, um einen gewünschten Herzrhythmus bereitzustellen oder wiederherzustellen; und
Mittel zum Integrieren des gesammelten geringsten abgeschätzten pulmonalarteriellen diastolischen Druckwerts (ePAD) mit bzw. in den Mitteln zum Überwachen und/oder Stimulieren von Herzgewebe, um ein atrioventrikuläres (AV) Stimulationsintervall für den Patienten zu optimieren.

2. System nach Anspruch 1, bei dem die hämodynamischen Überwachungsmittel einen der nachfolgenden Transducer aufweisen, wobei jeder von diesen ein Ausgabesignal bereitstellt, das ihn direkt oder indirekt wenigstens ein hämodynamisches Maß des Patienten anzeigt:
ein Absolutdrucksensor, der dafür eingerichtet ist, fluidisch mit einer Herzkammer des Patienten verbunden zu werden, ein Absolutdrucksensor, der dafür eingerichtet ist, fluidisch mit einer pulmonalen Arterie eines Patienten verbunden zu werden, ein Absolut- oder Differentialdrucksensor, der dafür eingerichtet ist, fluidisch mit einem Teil des Gefäßsystems eines Patienten verbunden zu werden.

3. System nach Anspruch 1, bei dem die Mittel zum Überwachen und/oder zum Stimulieren eines der folgenden aufweisen:
einen Pulsgenerator, einen implantierbaren Herzschrittmacher, einen implantierbaren Kardioverter-Defibrillator, eine Muskelstimulationsvorrichtung, einen externen Herzschrittmacher.

4. System nach Anspruch 3, bei dem die hämodynamischen Überwachungsmittel eines der folgenden aufweisen:
einen Absolutdrucksensor, der dafür eingerichtet ist, fluidisch mit einer Herzkammer des Patienten verbunden zu werden, einen Absolutdrucksensor, der dafür eingerichtet ist, fluidisch mit einer pulmonalen Arterie eines Patienten verbunden zu werden, einen Differentialdrucksensor, der dafür eingerichtet ist, fluidisch mit einem Abschnitt des Gefäßsystems eines Patienten verbunden zu werden, einen implantierbaren Absolutdrucksensor, der mit einem externen Referenzdrucksignal verbunden ist; und
wobei Aktivitätsniveaumessmittel optional mit dem Patienten verbunden sind und ein Ausgabesignal der Aktivitätsniveaumessmittel mit den hämodynamischen Überwachungsmitteln zeitsynchronisiert ist und die Aktivitätsniveaumessmittel von einem Accelerometer-Transducer oder einem piezoelektrischen Kristalltransducer abgeleitet sind.

5. Computerlesbares Medium, das Anweisungen aufweist, welche, wenn sie auf einem System nach einem der Ansprüche 1 bis 4 ausgeführt werden, das System dazu veranlassen, ein Verfahren zur Optimierung von Hämodynamiken eines Patienten unter Verwendung von von dem Patienten gesammelten hämodynamischen Daten auszuführen, mit:
Anweisungen zum Sammeln von hämodynamischen Daten, die aus einem niedrigsten eingeschätzten pulmonalarteriellen diastolischen Druckwert (ePAD) des Patienten bestehen, während einem Zeitabschnitt, in dem eine Herzfrequenz des Patienten aufgrund einer Aktivität des Patienten eine Ruhefrequenz überschreitet, wobei eine hämodynamische Überwachungseinrichtung dafür eingerichtet ist, in Fluidkontakt mit einem Volumen von venösem Blut des Patienten angeordnet zu werden;
Anweisungen zum Speichern der hämodynamischen Daten auf einem computerlesbaren Speichermedium;
Anweisungen zum Sammeln von Herzereignisdaten von dem Patienten;
Anweisungen zum Speichern der Herzereignisdaten auf einem computerlesbaren Speichermedium;
Anweisungen zum Analysieren des niedrigsten eingeschätzten pulmonalarteriellen diastolischen Druckwerts (ePAD) in Verbindung mit den Herzereignisdaten, um eine Herzstimulationssequenz zu bestimmen, die dafür vorgesehen ist, ein atrioventrikuläres (AV) Stimulationsintervall des Patienten zu optimieren; und
Anweisungen zum Bereitstellen der Herzstimulationssequenz an eine implantierbare Herzrhythmusstimulations- und/oder -überwachungsvorrichtung.

## Revendications

1. Système pour collecter des données hémodynamiques d'un patient et utiliser lesdites données pour optimiser un régime de stimulation cardiaque pour ledit patient,
**caractérisé par** :
des moyens de surveillance hémodynamique conçus pour collecter des données hémodynamiques pendant une période de temps au cours de laquelle une fréquence cardiaque du patient est élevée au-dessus d'une fréquence au repos du fait de l'activité dudit patient et pour mémoriser lesdites données hémodynamiques collectées, dans lequel lesdites données hémodynamiques sont constituées d'une valeur de pression diastolique d'artère pulmonaire estimée la plus faible (ePAD) ;
des moyens pour surveiller et/ou stimuler un tissu cardiaque d'un patient afin de délivrer ou de rétablir un rythme cardiaque voulu ; et
des moyens pour intégrer la valeur de pression diastolique d'artère pulmonaire estimée la plus faible (ePAD) aux moyens pour surveiller et/ou stimuler un tissu cardiaque afin d'optimiser un intervalle de stimulation atrio-ventriculaire (AV) pour ledit patient.

2. Système selon la revendication 1, dans lequel les moyens de surveillance hémodynamique comportent l'un des transducteurs suivants, chacun d'eux fournissant un signal de sortie directement ou indirectement indicatif d'au moins une mesure hémodynamique du patient :
un capteur de pression absolue adapté pour être couplé de manière fluide à une chambre cardiaque du patient, un capteur de pression absolue adapté pour être couplé de manière fluide à une artère pulmonaire d'un patient, un capteur de pression absolue ou différentielle adapté pour être couplé de manière fluide à une partie de la vasculature d'un patient.

3. Système selon la revendication 1, dans lequel les moyens de surveillance et/ou de stimulation comportent l'un des éléments suivants :
un générateur d'impulsions, un stimulateur cardiaque implantable, un défibrillateur cardioverteur implantable, un dispositif de stimulation musculaire, un stimulateur cardiaque externe.

4. Système selon la revendication 3, dans lequel les moyens de surveillance hémodynamique comportent l'un des éléments suivants :
un capteur de pression absolue adapté pour être couplé de manière fluide à une chambre cardiaque du patient, un capteur de pression absolue adapté pour être couplé de manière fluide à une artère pulmonaire d'un patient, un capteur de pression différentielle adapté pour être couplé de manière fluide à une partie de la vasculature d'un patient, un capteur de pression absolue implantable couplé à un signal de pression de référence externe ; et
dans lequel des moyens de mesure de niveau d'activité sont couplés de manière facultative audit patient et un signal de sortie desdits moyens de mesure de niveau d'activité est synchronisé dans le temps avec les moyens de surveillance hémodynamique et lesdits moyens de mesure de niveau d'activité sont dérivés d'un transducteur accéléromètre ou d'un transducteur à cristal piézo-électrique.

5. Support lisible par ordinateur contenant des instructions lesquelles, lorsqu'elles sont exécutées sur un système comme revendiqué dans l'une quelconque des revendications 1 à 4, amènent le système à mettre en oeuvre un procédé pour optimiser l'hémodynamique en utilisant des données hémodynamiques collectées depuis ledit patient, comportant :
des instructions pour collecter des données hémodynamiques constituées d'une valeur de pression diastolique d'artère pulmonaire estimée la plus faible (ePAD) depuis ledit patient, pendant une période de temps au cours de laquelle une fréquence cardiaque du patient est élevée au-dessus d'une fréquence au repos du fait de l'activité dudit patient, un moniteur hémodynamique étant adapté pour être disposé en contact fluide avec un volume de sang veineux dudit patient ;
des instructions pour mémoriser lesdites données hémodynamiques dans un support de mémoire lisible par ordinateur ;
des instructions pour collecter des données d'événement cardiaque depuis le patient ;
des instructions pour mémoriser les données d'événement cardiaque dans un support de mémoire lisible par ordinateur ;
des instructions pour analyser ladite valeur de pression diastolique d'artère pulmonaire estimée la plus faible (ePAD) en conjonction avec lesdites données d'événement cardiaque afin de déterminer une séquence de stimulation cardiaque destinée à optimiser un intervalle de stimulation atrio-ventriculaire (AV) dudit patient ; et
des instructions pour délivrer ladite séquence de stimulation cardiaque à un dispositif de surveillance et/ou de stimulation du rythme cardiaque implantable.
